# DEMANDE DE BREVET EUROPEEN

(11) **EP 1 502 583 A1**
(43) Date de publication de la demande: **02.02.2005**
(21) Numéro de dépôt: 04291718.7
(22) Date de dépôt: 07.07.2004
(51) Int. Cl.: A61K 7/48

(54) **Composition cosmétique comprenant un solvant volatile, une charge organique et une charge inorganique; et ses utilisations**

(30) Priorité: 01.08.2003 FR 0309564
(71) Demandeur: L'OREAL, 75008 Paris (FR)
(72) Inventeur: Ricard, Audrey, 75012 Paris (FR); Girier Dufournier, Franck, 75011 Paris (FR); Lemann, Patricia, 94000 Creteil (FR); Khachikian, Hélène, 94140 Alfortville (FR)
(74) Mandataire: Boulard, Denis

(57) **Abrégé**

La présente demande concerne des compositions cosmétiques, en particulier de maquillage ou de soin de la peau comprenant au moins une phase grasse comprenant au moins une huile volatile, au moins une charge organique et au moins une charge minérale, caractérisée en ce que ladite composition présente
- un indice de flou (Th-Td)/Th*100 supérieur ou égal à 40%, et
- un indice de transparence Th supérieur ou égal à 70%
Th étant la transmittance hémisphérique de ladite composition et Td étant la transmittance directe de ladite composition.

Cette composition est en particulier utile pour masquer les r ides e t autres d éfauts d u contour des lèvres. Elle permet également de modifier la perception visuelle du volume de la partie du corps sur laquelle elle est appliquée.

## Description

L'invention concerne une composition cosmétique, en particulier de maquillage et/ou de soin, particulièrement efficace pour diminuer ou supprimer les imperfections de la peau par un effet optique, en diffusant la lumière incidente dans plusieurs directions.

Ces compositions peuvent se présenter en particulier sous la forme d'un rouge à lèvres ou d'un fond de teint. Elles permettent notamment de masquer les rides et autres défauts du contour des lèvres. Elles permettent également de modifier la perception visuelle du volume de la partie du corps sur laquelle elles sont appliquées.

Les compositions de fonds de teint ont généralement pour objet d'unifier le teint : pour cela, on utilise des compositions dites matifiantes qui peuvent aider à estomper les défauts de la peau tels que les microreliefs, les rides, les ridules, les pores ou les variations de couleur de la peau. Ces compositions matifiantes donnent un aspect mat à la peau en diffusant la lumière à la surface de la peau.

Les compositions matifiantes classiques contiennent généralement assez peu de corps gras. Elles sont généralement constituées d'une haute proportion de poudres adsorbantes du sébum et d'huile excédentaire de la composition non-adsorbée par la peau. Parmi les poudres matifiantes d'origine naturelle ou synthétique, on peut citer notamment les charges telles que le talc, l'amidon, le mica , la silice, les poudres de nylon, les poudres de polyéthylène, la poly-beta-alanine, les poly(méthacrylate de méthyle). Ce type de charges présente l'inconvénient, à des fortes teneurs, de ne pas apporter à la peau un aspect naturel en donnant un dépôt poudreux voire plâtreux de couleur blanchâtre. De plus, les c ompositions les c ontenant s ont g énéralement d esséchantes à long t erme et s'étalent difficilement.

La présente invention concerne des compositions de maquillage ou de soin qui permettent de masquer visuellement les imperfections de la peau, par exemple les rides, les pores, des taches, une texture irrégulière, des différences de ton, des boutons. Les compositions de l'invention forment un film translucide sur la peau et laissent ainsi un aspect naturel à la peau maquillée.

Contrairement à certaines compositions de l'art antérieur, la composition de l'invention ne laisse pas de traces visibles sur la peau de couleur blanche ou teintée. Le consommateur n'a pas besoin de choisir une teinte de produit la plus proche de sa carnation car la composition de l'invention s'adapte à n'importe quel type de peau et procure un effet visuel immédiat d'amélioration de l'uniformité de la peau en masquent les défauts.

On a proposé dans le document US 6 511 672 d'associer des plaquettes d'alumine traitées avec de l'oxyde de fer et des plaquettes de mica enrobées de billes de silice, pour créer une mosaïque de couleur au niveau microscopique et diffuser la lumière de manière à faire un écran qui rendent les lignes, les rides, les déformations et les défauts de coloration de la peau moins visibles à l'oeil nu.

De même, le document US 6 174 533 décrit l'utilisation de dioxyde de titane, d'oxyde de zinc ou de dioxyde de zirconium de granulométrie comprise entre 100 et 300 nm et d'indice de réfraction au moins égale à 2, pour masquer les rides et d'autres états de surface irréguliers de la peau.

Il subsiste toujours le besoin de compositions cosmétiques permettant d'obtenir un teint, des lèvres, un contour des lèvres et plus généralement une peau uniforme, homogène, d'aspect naturel, lumineux, vivant, ces compositions présentant par ailleurs un bon confort après application sur la peau. On recherche également des compositions cosmétiques qui procurent un effet visuel plus flou pour mieux camoufler les microreliefs de la peau et ses disparités de teinte.

La Demanderesse a découvert de manière surprenante que l'association d'une huile volatile, d'une charge organique et d'une charge minérale permet d'obtenir des compositions cosmétiques qui masquent mieux les imperfections de la peau, et permettent de modifier la perception du volume de la partie du corps sur laquelle elles sont appliquées, tout en préservant un aspect naturel à la peau sur laquelle elles sont appliquées.

Selon un de ses aspects, l'invention a pour objet une composition cosmétique, en particulier de maquillage et/ou de soin, comprenant au moins une phase grasse contenant au moins une huile volatile, au moins une charge organique et au moins une charge minérale, caractérisée en ce que ladite composition présente
- un indice de flou (Th-Td)/Th*100 supérieur ou égal à 40%, et
- un indice de transparence Th supérieur ou égal à 70%
Th étant la transmittance hémisphérique de ladite composition et Td étant la transmittance directe de ladite composition.

Une telle composition confère au teint, aux lèvres et plus généralement la peau, une plus grande uniformité visuelle, une plus grande homogénéité, de la transparence et un teint lumineux. Appliquée sur les lèvres, la composition de l'invention permet de lisser les lèvres par effet optique, de masquer les rides et les imperfections des lèvres et du contour des lèvres.

Selon un autre aspect, l'invention a pour objet une composition cosmétique, en particulier de maquillage et/ou de soin, comprenant des particules de mica traitées en surface par de l'hydroxyde d'aluminium, ladite composition présentant
- un indice de flou (Th-Td)/Th*100 supérieur ou égal à 40%, et
- un indice de transparence Th supérieur ou égal à 70%
Th étant 1a transmittance hémisphérique de ladite composition et Td étant la transmittance directe de ladite composition.

Les particules de mica sont avantageusement en une quantité suffisante pour obtenir un indice de flou et un indice de transparence satisfaisant.

Selon un autre aspect, l'invention a pour objet une composition cosmétique, en particulier de maquillage et/ou de soin, comprenant des particules de mica enrobées ou non enrobées et des particules d'amidon modifié ou non modifié.

Par charges, il faut comprendre des particules colorées ou blanches, minérales ou organiques, lamellaires ou non lamellaires, solides à température ambiante (25°C) et pression atmosphérique, qui n'interagissent pas chimiquement avec les autres ingrédients de la composition et qui sont en particulier insolubles dans ces ingrédients même lorsque ces ingrédients sont portés à une température supérieure à la température ambiante. Les organopolysiloxanes élastomères réticulés tels que ceux décrits dans EP-A-0295886 et US 5.266.321 ne sont pas considérées comme des charges au sens de la présente invention.

Par charges organiques, on entend des charges constituées d'un unique composé chimique organique ou des charges constituées d'un substrat organique enrobée d'un autre composé organique ou minéral.

Par charges minérales, on entend des charges constituées d'un unique composé chimique minéral ou des charges constituées d'un substrat minéral enrobée d'un autre composé organique ou minéral.

L'indice de flou (Th-Td)/Th*100 peut être mesuré selon le protocole décrit ci après à l'aide d'un spectrophotomètre et d'une sphère d'intégration par exemple placée derrière l'échantillon.
- Th est la transmittance hémisphérique de la composition: elle est définie par le rapport entre l'intensité lumineuse reçue par la composition et l'intensité lumineuse restituée par la composition dans toutes les directions,
- Td est la transmittance directe de la composition: elle est définie par le rapport entre l'intensité lumineuse reçue par la composition et l'intensité lumineuse restituée par la composition dans le même axe.

Selon un mode de réalisation, Th et Td sont mesurées à l'aide d'un spectrophotomètre VARIAN Cary 300 et d'une sphère d'intégration de marque Lab sphere placée derrière l'échantillon, selon le protocole suivant. Un film de la composition selon l'invention, d'épaisseur 20 µm, est étalé sur une lame de quartz à creuset, puis placé 5 minutes à 37°C.

Pour mesurer Th, on utilise le spectrophotomètre en mode de transmission diffuse, à une longueur d'onde variant de 400 à 700 nm. On se place en mode transmission %T, à une vitesse de balayage à 240 nm/min, en « double reverse ». On fait une correction de la ligne de base en faisant la mesure d'une lame vide de référence. Ceci donne la valeur maximale de l'intensité qui peut être transmise. On place la lame de quartz contenant le film de composition dans le compartiment de mesure et on mesure Th.

Td est mesurée à l'aide du même spectrophotomètre selon le protocole suivant : on utilise le spectrophotomètre en mode de transmission directe, à une longueur d'onde variant de 400 à 700 nm. On se place en mode transmission %T, à une vitesse de balayage 240 nm/min, en mode double. On place une lame de quartz vide dans le compartiment de référence et la lame de quartz contenant l'échantillon dans le compartiment de mesure. On mesure Td.

Les valeurs de Th et Td sont les moyennes de chacune des valeurs spectrales mesurées.

Plus la valeur de (Th-Td)/Th*100 est élevée, meilleur est l'effet flou. Plus la valeur de Td est élevée, plus la composition est transparente.

L'indice de flou est supérieur ou égal à 40%, de préférence supérieur ou égal à 50%.

L'indice de transparence est avantageusement supérieur ou égal à 85%, de préférence supérieur ou égal à 95%.

De préférence, la charge organique et la charge minérale de l'invention ont indépendamment l'une de l'autre une granulométrie moyenne de particules inférieure ou égale à 50 microns, de préférence 30 microns, de préférence encore 15 microns, et préférentiellement inférieure ou égale 10 microns, par exemple de l'ordre de 5 ou 3 microns.

On peut ainsi obtenir une composition qui comprend peu de pigments, voire aucun pigment mais qui camoufle bien les micro reliefs et autres imperfections de la peau. Selon un mode réalisation, la composition de l'invention est exempte de pigments ou de nacres.

La charge minérale est constituée ou comprend un matériau choisi par exemple parmi le talc, le mica, la silice, le kaolin, les microsphères de silice creuses, les microcapsules de verre, les oxydes de titane, de fer et de zinc. La charge minérale peut éventuellement être enrobée et être notamment constituée d'un substrat de mica enrobé d'alumine, de dioxyde de titane, de silice, d'oxyde d'aluminium et/ou de sulfate de barium, telle que le composé TiO2/silice, le composé mica/TiO2, le composé silice/TiO2, et le complexe de silice et de TiO2/TiO2 . La charge lamellaire peut être de forme lamellaire ou non lamellaire.

La charge minérale est de préférence choisie parmi la silice, le talc, le mica et les composites contenant du mica, les composites de silice et de dioxyde de titane, les composites de silice et d'oxyde de zinc, et leurs mélanges.

Parmi les charges minérales convenant particulièrement bien à l'invention, on peut citer :
- l'oxyde de titane recouvert de silice tel que Flonac TS 40 C distribué par Eckart,
- des microbilles de silice de granulométrie comprise entre 3 et 15 microns telles que Silica Beads SB 150 fabriquées par Miyoshi, ou des microbilles de silice Sunsphere H31 de granulométrie égales à 3 microns fabriquées par Asahi Glass,
- des plaquettes de silice de granulométrie égale à 1,5 micron, par exemple Chemiceler ou Finesil F-80 distribuées par Sumitomo,
- des plaquettes de mica recouvertes de micro-billes d'hydroxyde d'aluminium dans un rapport pondéral de 60/40 ou de 65/35, notamment le produit Excel Mica JP-1 ou le produit Excel Mica JP-2 commercialisés par Miyoshi,
- de la silice enrobée de dioxyde de titane et de silice poreuse, de granulométrie par exemple égale à 0,6 micros, comme le produit ACS-0050510 de Catalysts et Chemicals tel que la proportion silice/dioxyde de titane/silice poreuse est égale à 85/5/10,
- du mica recouvert de sulfate de baryum et d'oxyde de titane, comme le produit Naturaleaf de Merck tel que la proportion mica/sulfate de baryum/oxyde de titane est égale à 66/22/12,
- un complexe de silice et d'oxyde de cérium enrobé de silice amorphe, de granulométrie comprise entre 1 et 10 microns, comme le produit Ceriguard SC,
- un complexe de silice et d'oxyde de titane enrobé de polyhydrogénométhylsiloxane, par exemple dans les proportions 93/5/2,
- des plaquettes de séricite recouvertes d'oxyde de titane, d'alumine et de silice, comme par exemple le Coverleaf AR-20121 M commercialisé par Catalysts et Chemicals dont la granulométrie est comprise entre 5 et 10 µm et telle que la proportion séricite/oxyde de titane/alumine/silice est égale à 67/5/18/10.

Dans une forme particulièrement avantageuse de réalisation de l'invention, la charge minérale est choisie parmi les composites du mica et d'un hydroxyde métallique. De préférence, la charge minérale est constituée de mica recouvert de micro-billes d'hydroxyde d'aluminium, cette charge ayant une taille moyenne des particules égales à 10 microns (plus ou moins 2 microns). La taille moyenne de particules (ou granulométrie) est définie dans toute la demande comme la taille donnée par la distribution granulométrie statistique à la moitié de la population, dite D50.

De préférence, la charge minérale est présente dans la composition à une teneur allant avantageusement de 0,1 à 40 %, de préférence encore de 3 à 15%, de préférence encore de 2% à 8%, en poids par rapport au poids total de la composition, de préférence de l'ordre de 5 %.

Parmi les charges organiques convenant à l'invention, on peut citer les poudres de polyamide comme le Nylon ®, les poudres de poly-β-alamine, les poudres de polyéthylène, les poudres de polytétrafluoroéthylène, la lauroyl-lysine, l'amidon, les microsphères creuses polymériques telles que celles de chlorure de polyvinylidène/acrylonitrile comme l'Expancel ®, les copolymères d'acide acrylique comme le Polytrap ®, les microbilles de résine de silicone, par exemple le Topspearl.

Selon un mode de réalisation préféré, la charge organique est de l'amidon, en particulier de l'amidon modifié, comme par exemple de l'amidon réticulé par l'anhydride octénylsuccinique commercialisé sous la référence Dry Flo Plus (28-1160) par National Starch.

De préférence, la charge organique est présente dans la composition à une teneur allant avantageusement de 0,1 à 40 %, de préférence encore de 3 à 15%, de préférence encore de 2% à 8%, en poids par rapport au poids total de la composition, de préférence de l'ordre de 5 %.

La charge organique et la charge minérale ont de préférence un indice de réfraction supérieur à 1, de préférence allant de 1,25 et 1,9, plus préférentiellement allant de 1,45 et 1,55.

La charge minérale et la charge organique sont de référence dans un rapport massique compris entre 1 :1 et 1 :3.

Selon un mode mise en oeuvre, la charge minérale est lamellaire lorsque la charge organique est sphérique et vice versa. Par l'expression "sous f orme lamellaire", o n entend sous forme de plaquettes, on désigne des particules dont le rapport entre la plus grande dimension et l'épaisseur est supérieur ou égal à 5, voire 10 ou mieux 20. Par l'expression "sphérique", on entend une forme essentiellement sphérique, notamment sous la forme de billes, de préférence de granulométrie comprise entre 1 et 15 microns de préférence de l'ordre de 10 microns.

La composition selon l'invention contient avantageusement moins de 30% de charges, de préférence moins de 20% de charges.

Selon un autre de ses aspects, l'invention a pour objet une composition cosmétique, en particulier de maquillage et/ou de soin, comprenant une charge minérale choisie parmi les composites d'un silicate inorganique et d'un hydroxyde métallique.

Dans ce mode de mise en oeuvre, la composition présente
- un indice de flou (Th-Td)/Th*100 supérieur ou égal à 40%, et
- un indice de transparence Th supérieur ou égal à 70%
Th é tant la t ransmittance h émisphérique d e ladite c omposition e t Td é tant la transmittance directe de ladite composition.

Le silicate inorganique est de préférence du mica naturel ou synthétique. Le mica peut être choisi parmi les micas muscovite, phlogopite, tiotite, sericite, lepidolite, paragonite, les micas synthétiques et leurs mélanges.

L'hydroxyde métallique est un hydroxyde d'un métal choisi parmi Ag, Au, Cu, Al, Ni, Sn, Mg, Cr, Mo, Ti, Pt, Va, Rb, W, Zn, Ge, Te, Se et leurs alliages. On préfère l'hydroxyde d'aluminium comme hydroxyde métallique.

Selon un mode de mise en oeuvre, la charge minérale est un composite constituée de particules de silicate inorganique recouvertes en surface par l'hydroxyde métallique. Le silicate inorganique est avantageusement sous forme lamellaire ou sous forme de plaquettes. Par l'expression « sous forme de plaquettes », on désigne des particules dont le rapport entre la plus grande dimension et l'épaisseur est supérieur ou égal à 5, voire 10 ou mieux 20.

L'hydroxyde métallique est avantageusement sous forme essentiellement sphérique, notamment sous la forme de billes, de préférence de granulométrie comprise entre 1 et 15 microns de préférence de l'ordre de 10 microns.

La proportion massique entre le silicate inorganique et l'hydroxyde métallique est de préférence comprise entre 50/50 et 80/20, de préférence entre 60/40 et 70/30, de préférence égale à environ 65/35.

Les billes d'hydroxyde métallique sont avantageusement des billes d'hydroxyde d'aluminium. Selon cet aspect de l'invention, la composition contient avantageusement de l'amidon éventuellement modifié.

Selon un mode de mise en oeuvre, la composition comprend une huile non volatile.

Par "huile volatile", on entend tout milieu non aqueux susceptible de s'évaporer au contact de la peau, à température ambiante (25°C) et pression atmosphérique (760 mm de Hg). Le ou les huiles cosmétiques volatiles, liquides à température ambiante, ont notamment une pression de vapeur, mesurée à température ambiante et pression atmosphérique, allant de 10⁻³ à 300mm de Hg (0,266 Pa à 40 000 Pa), de préférence de 0,02 mm à 300mm de Hg (2,66 Pa à 40 000 Pa) et mieux allant de 0,1 à 90 mm de Hg (13 Pa à 12 000 Pa).

Selon l'invention, ces huiles volatiles peuvent être des huiles hydrocarbonées, des huiles siliconés comportant éventuellement des groupements alkyle ou alkoxy pendants ou en bout de chaîne siliconée ou un mélange de ces huiles.

De préférence, les huiles volatiles sont des huiles cosmétiques choisies parmi les huiles n'ayant pas de point éclair, les huiles ayant un point éclair allant de 40°C à 100°C, et leurs mélanges, en vue de faciliter leur mise en oeuvre. En outre, ils présentent avantageusement une température d'ébullition à pression atmosphérique inférieure à 220°C et mieux inférieure à 210°C, notamment allant de 110 à 210°C. De préférence, ces huiles volatiles ne sont pas des monoalcools à au moins 7 atomes de carbone.

Comme huile volatile utilisable dans l'invention, on peut citer les huiles de silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclo-tétrasiloxane, le décaméthylcyclopentasiloxane, le dodécaméthylcyclohexasiloxane, l'heptaméthyl hexyltrisiloxane, l'heptaméthyloctyl trisiloxane, l'hexaméthyldisiloxane, l'octaméthyl trisiloxane, le décaméthyl tétrasiloxane, le dodécaméthyl pentasiloxane et leurs mélanges.

Comme autre huile volatile utilisable dans l'invention, on peut citer les huiles volatiles hydrocarbonées ayant de 8 à 16 atomes de carbone et leurs mélanges et notamment les alcanes ramifiés en C₈-C₁₆ comme les isoalcanes (appelés aussi isoparaffines) en C₈-C₁₆, l'isododécane, l'isodécane, l'isohexadécane et par exemple les huiles vendues sous les noms commerciaux d'Isopars' ou de Permetyls, les esters ramifiés en C₈-C₁₆ comme le néo pentanoate d'iso-hexyle et leurs mélanges.

De préférence, on utilise l'isododécane (Permetyls 99 A), les isoparaffines en C₈-C₁₆ comme l'Isopar L, E, G ou H, leurs mélanges, éventuellement associés au décaméthyl tétrasiloxane ou au cyclopentasiloxane.

On peut aussi utiliser des huiles volatiles fluorées.

Ces huiles volatiles représentent notamment de 5 à 80 % du poids total de la composition, et mieux de 10 à 30 %.

La composition selon l'invention peut comprendre au moins une huile non volatile.

Par huile non volatile, on entend un corps gras liquide à température ambiante (20°C) et qui ne s'évapore pas à cette même température.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment les phényltriméthicones,
- les huiles d'origine végétale ou minérale, telles que l'huile de paraffine, de vaseline, le perhydrosqualène, la lanoline, l'huile d'abricot, l'huile de germes de blé, d'amande douce, de calophyllum, de sésame, de macadamia, de pépins de raisin, de colza, de coprah, d'arachide, de tournesol, de palme, de ricin, d'avocat, de jojoba, d'olive ou de germes de céréales, la fraction liquide du beurre de karité; des esters d'acides gras et de polyol, en particulier les triglycérides liquides, le myristate d'isopropyle, l'huile de mygliol, le néopentanoate d'isostéaryle, le palmitate d'éthyl-2-hexyle, l'huile de ricin, l'acétyl-citrate de tributyle; des alcools, en particulier l'octyl-2-dodecanol ; des acétylglycérides ; des octanoates, décanoates ou ricinoléates d'alcools ou de polyalcools ; des triglycérides d'acides gras; des glycérides, les huiles fluorées et perfluorées ;
- les composés amidés, en particulier ceux décrits dans la demande PCT/FR98/01077 comme le N-néopentanoyl-2-octyl-dodécylamine, le N-néopentanoyl-2-butyl-octylamine, le N-(3,5,5-triméthyl-hexanoyl)-2-octyl-dodécylamine, le N-(3,5,5-triméthyl-hexanoyl)-2-butyl-octylamine,
- leurs mélanges.

De préférence, l'huile non volatile de la phase grasse de la composition selon l'invention comprend une ou plusieurs huiles choisies parmi le polyisobutène hydrogéné, le propionate d'arachidyle, le néopentanoate d'octydodécyle, le polybutène, les diméthicones, l'octyldodécanol, et leurs mélanges.

De préférence, l'huile non volatile représente de 1 à 85%, de préférence encore de 5% à 60%, de préférence encore de 10% à 50% de préférence encore de 20% à 40%en poids, par rapport au poids total de la composition.

La composition peut comprendre une cire et/ou un pâteux, en particulier lorsqu'elle de présente sous la forme d'un stick.

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide. Le composé pâteux au sens de l'invention a avantageusement une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi
- la lanoline et ses dérivés
- les composés siliconés polymères ou non
- les composés fluorés polymères ou non
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters,
et leurs mélanges.

Le composé pâteux représente de préférence 1 à 99%, mieux 1 à 60%, mieux 2 à 30% et mieux encore 5 à 15% en poids de la composition.

Parmi les cires, on peut notamment citer :
- les cires d'origine animale, végétale, minérale ou synthétique telles que les cires microcristallines, la paraffine, le pétrolatum, la vaseline, l'ozokérite, la cire de Montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25 °C, les ozokérites, les esters gras et les glycérides concrets à 25 °C; les cires de polyéthylène et les cires obtenues par synthèse de Fischer-Tropsch; des huiles hydrogénées concrètes à 25 °C; des lanolines; des esters gras concrets à 25 °C; les cires de silicone; les cires fluorées; leurs mélanges.

La composition selon l'invention peut contenir d'autres charges que celles décrites précédemment.

Ces charges peuvent être présentes dans la composition à raison de 0-20 % en poids, de préférence de 2-15 % en poids, par rapport au poids total de la composition. La composition est de préférence exempte de telles charges.

Les compositions selon l'invention peuvent également comprendre des colorants comme par exemple les colorants hydrosolubles ou liposolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le Rouge Soudan III (nom CTFA D&C red 17), la lutéine, le vert de quinizarine (nom CTFA DC green 6), le pourpre d'Alizurol SS (nom CTFA DC violet N°2), les dérivés caroténoïdes comme le lycopène, le béta-carotène, la bixine, la capsantéine, et/ou leurs mélanges.

Les compositions de l'invention comprennent un milieu cosmétiquement et/ou physiologiquement acceptable, c'est-à-dire un milieu compatible avec la peau, autrement dit toute zone cutanée du corps et du visage.

Une telle composition se présente généralement sous forme fluide ou solide. Par suite une telle composition peut se présenter sous forme fluide, sous forme pâteuse, sous forme semi-solide, et sous forme solide, généralement en stick. Une telle composition peut se présenter sous la forme d'un fond de teint, par exemple sous forme fluide, en pot ou en stick, de laque à lèvres, typiquement sous forme pâteuse, ou de rouges à lèvres, typiquement sous forme semi-solide (« gloss » en terminologie anglo-saxonne), ou de rouges à lèvres sous forme solide, en stick.

La composition se présente de préférence sous la forme d'un stick de maquillage, comme un stick de fard à paupières, un stick de fond de teint, un stick anti-cernes ou un stick de rouge à lèvres.

La composition selon l'invention peut éventuellement comprendre une phase aqueuse qui peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale.

Les compositions de l'invention peuvent ainsi se présenter sous la forme d'une composition anhydre ou d'une émulsion huile-dans-eau (H/E), une émulsion eau-dans-huile (E/H), une émulsion multiple ou une solution multiphasée. Elles peuvent également se présenter sous la forme d'une dispersion vésiculaire, par exemple sous la forme d'une phase huileuse dispersée dans une phase aqueuse et stabilisée par des liposomes.

Dans le cas où la composition selon l'invention est une émulsion, elle peut comprendre des c omposés a mphiphiles c'est-à-dire d es c omposés c omprenant à la fois u ne p artie lipophile (partie apolaire) et une partie hydrophile (partie polaire) et pouvant s'adsorber à une surface ou une interface. De tels composés sont par exemple les tensioactifs et les co-tensioactifs.

Comme tensioactif H/E, on peut citer notamment (CTFA) : l'acide stéarique combiné à la triéthanolamine, le mélange de mono et distéarate de glycérol, le lauroyl sarcosinate de sodium, le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate. Comme tensioactif E/H, on peut citer notamment le mélange p olyglycéryl-4 i sostéarate/ cétyldiméthicone copolyol/ hexyl laurate et le mélange Mineral oil/petrolatum/ozokérite/glycéryl oléate/alcool de lanoline.

Lorsque la composition selon l'invention comprend des tensio-actifs, ceux-ci font partie de la fraction non volatile de la phase grasse et sont donc pris en compte dans le calcul de n₁.

De préférence, la composition selon l'invention se présente sous la forme d'une émulsion E/H.

La composition selon l'invention peut comprendre en outre tout additif usuellement utilisé dans le domaine cosmétique, tels que des antioxydants, des filtres UV, des colorants, des parfums, des huiles essentielles, des conservateurs, des actifs cosmétiques, des hydratants, des vitamines, des sphingolipides, des polymères liposolubles notamment hydrocarbonés, tels que le polybutène, les polyalkylènes, les p olyacrylates e t les polymères siliconés compatibles avec les corps gras, et/ou leurs mélanges.

Ces additifs peuvent être présents dans la composition à raison de 0-15% en poids, par rapport au poids total de la composition.

Selon un mode de mise en oeuvre, la composition de l'invention est anhydre; elle contient moins de 5% d'eau ajoutée au cours du procédé de préparation, présente dans la composition à titre d'impureté ou adsorbée par la composition. De préférence; la composition anhydre contient moins de 3% d'eau, voire même moins de 1% d'eau.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme d'un produit cosmétique et notamment sous la forme d'un produit de maquillage, en particulier un fond de teint, un anticerne, un produit pour les lèvres, un produit de maquillage pour le corps ou bien encore d'un produit de soin pour le corps et/ou le visage.

Les compositions selon l'invention peuvent être préparées selon les méthodes de préparation classiques bien connues de l'homme du métier.

La présente invention a encore pour objet l'utilisation d'au moins une charge minérale, d'au moins une charge organique et d'au moins une huile volatile dans une composition cosmétique pour masquer les imperfections de la peau, et/ou de modifier la perception du volume de la partie du corps sur laquelle la composition est appliquée, tout en préservant un aspect naturel à la peau sur laquelle elle est appliquée.

Les exemples suivants illustrent l'invention. Dans ces exemples, les proportions indiquées sont des pourcentages en poids.

### EXEMPLE 1 : Composition lissante pour les lèvres

| | |
|---|---|
| Polyisobutène hydrogéné (Nof Corp.) | 10 % |
| propionate d'arachidyle (Alzo) | 1,8 % |
| néopentanoate d'octydodécyle (Bernel) | 1,3 % |
| disteardinonium hectorite (Elementis) | 0,3 % |
| mica recouvert de microbilles d'hydroxyde d'aluminium (Excel Mica JP-2 ® de Miyoshi) | 5 % |
| amidon réticulé par l'anhydride octényl succinique (Dry Flo Plus (28-1160) ® de National Starch) | 10% |
| polybutène | 4,7 % |
| cire de polyéthylène | 15,8 % |
| diméthicone (DC 200-5 cSt) | 17,3% |
| décaméthyl tétrasiloxane (DC 200-1,5 cSt) | 22 % |
| octyldodécanol | 3,2 % |
| cire de lanoline oxypropylénée (50P) (Cognis) | 8,4 % |

## Revendications

1. Composition cosmétique, en particulier de maquillage et/ou de soin, comprenant au moins une phase grasse comprenant au moins une huile volatile, au moins une charge organique et au moins une charge minérale, **caractérisée en ce que** ladite composition présente
• un indice de flou (Th-Td)/Th*100 supérieur ou égal à 40%, et
• un indice de transparence Th supérieur ou égal à 70%
Th étant la transmittance hémisphérique de ladite composition et Td étant la transmittance directe de ladite composition.

2. Composition selon la revendication 1, **caractérisée en ce qu'**elle est anhydre.

3. Composition selon la revendication 1 à 2, **caractérisée en ce qu'**elle est sous la forme d'un stick de maquillage, comme un stick de fard à paupières, un stick de fond de teint, un stick anti-cernes ou un stick de rouge à lèvres.

4. Composition selon l'une des revendications 1 à 3, **caractérisée en ce que** l'indice de flou est supérieur ou égal à 50%.

5. Composition selon la revendication 1 ou 2, **caractérisée en ce que** l'indice de transparence est supérieur ou égal à 85%, de préférence supérieur ou égal à 95%.

6. Composition selon la revendication 1, **caractérisée en ce qu'**elle comprend la phase grasse comprend au moins une huile non volatile.

7. Composition selon l'une quelconque des revendications précédentes, **caractérisée en ce que** la charge minérale et la charge organique ont indépendamment l'une de l'autre une taille moyenne de particules inférieure ou égale à 50 microns, de préférence 30 microns, de préférence encore 15 microns, et préférentiellement inférieure ou égale 10 microns.

8. Composition selon l'une quelconque des revendications précédentes, **caractérisée en ce que** la charge minérale est lamellaire lorsque la charge organique est sphérique et vice versa.

9. Composition selon l'une quelconque des revendications précédentes, **caractérisée en ce que** la charge minérale est choisie parmi la silice, le talc, le mica et les composites contenant du mica, les composites de silice et de dioxyde de titane, les composites de silice et d'oxyde de zinc, et leurs mélanges.

10. Composition selon l'une quelconque des revendications précédentes, **caractérisée en ce que** la charge minérale est choisie parmi:
- l'oxyde de titane recouvert de silice,
- des microbilles de silice de granulométrie comprises entre 3 et 15 microns,
- des plaquettes de silice, de granulométrie égale à 1,5 micron,
- de la silice enrobée de dioxyde de titane et de silice poreuse, de granulométrie par exemple égale à 0,6 microns, par exemple telle que la proportion silice/dioxyde de titane/silice poreuse est égale à 85/5/10,
- du mica recouvert de sulfate de baryum et d'oxyde de titane, par exemple tel que la proportion mica/sulfate de baryum/oxyde de titane est égale à 66/22/12,
- un complexe de silice et d'oxyde de cérium enrobé de silice amorphe, de granulométrie comprise entre 1 et 10 microns,
- un complexe de silice et d'oxyde de titane enrobé de polyhydrogénométhylsiloxane,
- des plaquettes de séricite recouvertes d'oxyde de titane, d'alumine et de silice,
- et leurs mélanges.

11. Composition selon l'une quelconque des revendications 1 à 9, **caractérisée en ce que** la charge minérale est choisie parmi les composites du mica et d'un hydroxyde métallique, comme l'hydroxyde d'aluminium.

12. Composition selon l'une quelconque des revendications précédentes, **caractérisée en ce que** la charge organique est choisie parmi les poudres de polyéthylène, les poudres d'amidon, les poudres d'amidon modifié, comme l'amidon réticulé par l'anhydride octénylsuccinique, les poudres de nylon, les poudres de copolymères styrène/acrylique et leurs mélanges.

13. Composition selon l'une quelconque des revendications précédentes telle que la charge minérale est du mica ou un composite de mica de taille moyenne des particules inférieure ou égale à 50, de préférence inférieur à 10 microns, de préférence encore inférieur à 5 microns.

14. Composition selon l'une quelconque des revendications précédentes telle que la charge organique est de l'amidon de taille moyenne des particules inférieure ou égale à 50, de préférence inférieur à 10 microns, de préférence encore inférieur à 5 microns.

15. Composition selon l'une quelconque des revendications précédentes telle que la charge organique représente de 0,1 à 20%, de préférence encore de 8% à 15%, en poids par rapport au poids total de la composition.

16. Composition selon l'une quelconque des revendications précédentes telle que la charge minérale représente de 0,1 à 15%, de préférence encore de 1% à 10%, en poids par rapport au poids total de la composition.

17. Composition selon la revendication 4 telle que l'huile non volatile est choisie parmi le polyisobutène hydrogéné, le propionate d'arachidyle, le néopentanoate d'octydodécyle, le polybutène, les diméthicones, l'octyldodécanol, et leurs mélanges.

18. Composition selon l'une quelconque des revendications précédentes telle que l'huile non volatile représente de 1 à 85%, de préférence encore de 5% à 60%, de préférence encore de 10% à 50%, de préférence encore de 20% à 40% en poids, par rapport au poids total de la composition.

19. Composition selon la revendication 1 telle qu'elle comprend en outre une phase aqueuse.

20. Composition la revendication 1, **caractérisée en ce qu'**elle se présente sous la forme d'un produit cosmétique et notamment sous la forme d'un produit de maquillage, en particulier un fond de teint, un anticerne, un produit de maquillage pour le corps ou bien encore d'un produit de soin pour le corps et/ou le visage.

21. Composition selon l'une quelconque des revendications précédentes se présentant sous forme fluide ou solide.

22. Composition selon l'une quelconque des revendications précédentes se présentant forme fluide, sous forme pâteuse, sous forme semi-solide, et sous forme solide.

23. Utilisation d'au moins une charge minérale et d'au moins une charge organique dans une composition comprenant au moins une huile volatile dans le but a) de masquer les rides et autres défauts de la peau du visage ou du contour des lèvres, et/ou b) de modifier la perception visuelle du volume de la partie du corps sur laquelle la composition est appliquée.

24. Composition cosmétique, en particulier de maquillage et/ou de soin, comprenant une charge minérale, **caractérisée en ce que** ladite charge est choisie parmi les composites d'un silicate inorganique et d'un hydroxyde métallique.

25. Composition selon la revendication 24, **caractérisée en ce que** le silicate inorganique est du mica naturel ou synthétique.

26. Composition selon la revendication 24 ou 25, **caractérisée en ce que** l'hydroxyde métallique est l'hydroxyde d'aluminium.

27. Composition selon l'une des revendications 24 à 2 6, **caractérisée en ce que** la charge minérale est constituée de particules de silicate inorganique recouvertes en surface par un hydroxyde métallique.

28. Composition selon la revendication précédente, **caractérisée en ce que** le silicate inorganique est sous forme lamellaire.

29. Composition selon l'une des revendications 24 à 28, **caractérisée en ce que** l'hydroxyde métallique est sous forme de billes, de préférence de granulométrie comprise entre 1 et 15 microns de préférence de l'ordre de 10 microns.

30. Composition selon l'une des revendications 24 à 29, **caractérisée en ce que** la proportion massique entre le silicate inorganique et l'hydroxyde métallique est comprise entre 50/50 et 80/20, de préférence entre 60/40 et 70/30, de préférence égale à environ 65/35.

31. Composition selon la revendication 24, **caractérisée en ce que** ladite composition présente
• un indice de flou (Th-Td)/Th*100 supérieur ou égal à 40%, et
• un indice de transparence Th supérieur ou égal à 70%
Th étant la transmittance hémisphérique de ladite composition et Td étant la transmittance directe de ladite composition.

32. Composition selon la revendication 24, **caractérisée en ce qu'**elle contient en outre de l'amidon éventuellement modifié.
